# EUROPEAN PATENT APPLICATION

(11) **EP 3 434 199 A1**
(43) Date of publication of application: **30.01.2019**
(21) Application number: 17770399.8
(22) Date of filing: 24.03.2017
(51) Int. Cl.: A61B 17/062, A61B 17/06

(54) **SUTURING DEVICE**

(30) Priority: 24.03.2016 JP 2016060880
(71) Applicant: National University Corporation Kagawa University, Takamatsu-shi, Kagawa 760-8521 (JP); Zeon Corporation, Tokyo 100-8246 (JP)
(72) Inventor: MORI, Hirohito, Kita-gun Kagawa 761-0793 (JP); INOUE, Kouichi, Tokyo 100-8246 (JP)
(74) Representative: Plougmann Vingtoft a/s
(86) International application number: PCT/JP2017/011974
(87) International publication number: WO 2017/164359

(57) **Abstract**

Provided are a front arm (11), a rear arm (12), and arm moving means (13). The rear arm (12) includes a suture needle part (14) having a pair of needle-like members (15 and 16) each provided so as to have a tip portion facing the front arm (11) and a central axis parallel to a direction in which the rear arm (12) and the front arm (11) are moved toward and away from each other. The front arm (11) includes an accommodating portion (17) provided with a pair of accommodating spaces (18 and 19) capable of accommodating the corresponding tip portions of the pair of needle-like members (15 and 16) when the front arm (11) and the rear arm (12) are moved toward each other. The pair of accommodating spaces (18 and 19) of the accommodating portion (17) accommodates a respective pair of engaging members (21 and 21) connected to each other with a suture thread (22) .

## Description

### TECHNICAL FIELD

The present invention relates to a suturing device. More specifically, the present invention relates to a suturing device attached to an endoscope, being used for suturing tissue in a gastrointestinal tract.

### BACKGROUND ART

Conventionally, when a tumor or the like is formed in a gastrointestinal tract such as a stomach, the tumor or the like is typically removed by a surgical operation. In recent years, surgery using a laparoscope has also been performed as a minimally invasive surgery that can reduce a burden on a patient as well as scars formed on a body surface compared to a surgical operation.

Recently, surgery using a flexible endoscope has been performed as less invasive surgery than surgery using a laparoscope. When cancer is early gastric cancer or the like, remaining in a mucous layer, the cancer can be excised with a flexible endoscope by peeling a submucosal layer including the mucous layer from a lumen of the gastrointestinal tract. This case allows surgery to be performed without leaving any scar on a body surface, so that not only a burden on the patient is small, but also recovery from the surgery is fast.

On the other hand, when a tumor is developed below the submucosal layer formed in the stomach wall, or the tumor reaches the muscularis propria, the muscularis propria also needs to be removed. In this case, an opening is formed in the stomach wall when the tumor is removed, so that the opening needs to be closed (sutured) after the tumor is removed. However, only a flexible endoscope cannot close the opening, so that surgery using the flexible endoscope and a laparoscope is also performed. Specifically, the tumor on the stomach wall is excised with the flexible endoscope, and surgery for suturing the opening formed after the tumor is removed is performed using the laparoscope.

While surgery using a flexible endoscope and a laparoscope is less invasive than a surgical operation, a hole for inserting the laparoscope into an abdominal cavity still needs to be formed. If a flexible endoscope enables not only resection of a tumor from a gastrointestinal lumen but also suturing, surgery can be performed without forming a scar on a body surface. For this reason, a suturing instrument for suturing an opening from the inside of a gastrointestinal lumen is currently being developed (e.g., Patent Document 1).

By using the suturing instrument disclosed in Patent Document 1, end faces of an opening formed when a tumor is excised can be brought into contact with each other to achieve a suture similar to that of surgical suturing. In addition, by using the suturing instrument disclosed in Patent Document 1, not only an opening through which a tumor is excised but also various holes or the like formed in a gastrointestinal tract can be sutured and closed.

### PRIOR ART DOCUMENT

### PATENT DOCUMENT

Patent Document 1: Japanese Patent No. 5294181

### SUMMARY OF THE INVENTION

### PROBLEMS TO BE SOLVED BY THE INVENTION

When tissue is excised from a stomach wall or the like, the tissue may be discharged into an abdominal cavity through an opening formed in the excised portion. When the excised tissue is cancer and the cancer tissue leaks into the abdominal cavity, the cancer may transfer to other organs and the like. If tissue such as cancer can be excised without forming an opening in a stomach wall or the like, the tissue can be excised from a gastrointestinal lumen more securely with a flexible endoscope. Thus, it is desired to develop a device that achieves the method described above.

In light of the above-mentioned circumstances, it is an object of the present invention to provide a suturing device capable of more easily suturing tissue from inside a gastrointestinal tract.

### MEANS FOR SOLVING THE PROBLEMS

A suturing device according to a first aspect of the present invention includes a front arm, a rear arm provided so as to be movable toward and away from the front arm, and arm moving means for moving the front arm and the rear arm toward and away from each other. The front arm and the rear arm each has a proximal end connected to the arm moving means . The rear arm includes a suture needle part having a pair of needle-like members each provided so as to have a tip portion facing the front arm and a central axis parallel to a direction in which the rear arm and the front arm are moved toward and away from each other. The front arm includes an accommodating portion provided with a pair of accommodating spaces capable of accommodating the corresponding tip portions of the pair of needle-like members when the front arm and the rear arm are moved toward each other. The pair of accommodating spaces of the accommodating portion accommodates a respective pair of engaging members connected to each other with a suture thread and engageable with the corresponding needle-like members. In the first aspect of the present invention, a suturing device according to a second aspect of the present invention is configured such that a distance from each of positions at which the respective front arm and rear arm are connected to the arm moving means to the pair of needle-like members is 5 mm or more, the distance being along a face perpendicular to a direction in which the rear arm and the front arm are moved toward and away from each other.

In the first or second aspect of the present invention, a suturing device according to a third aspect of the present invention is configured such that the suture needle part includes the pair of needle-like members provided side by side parallel to an axis of the rear arm, and the accommodating portion includes the pair of accommodating spaces provided side by side parallel to an axis of the front arm.

In the first, second, or third aspect of the present invention, a suturing device according to a fourth aspect of the present invention is configured such that the rear arm includes a plurality of the suture needle parts provided side by side parallel to the axis of the rear arm, and the front arm includes a plurality of the accommodating portions corresponding to the respective suture needle parts, the plurality of accommodating portions being provided side by side parallel to the axis of the front arm.

In the first, second, third, or fourth aspect of the present invention, a suturing device according to a fifth aspect of the present invention is configured to include a connecting mechanism that connects the rear arm and the front arm while allowing the rear arm and the front arm to move toward and away from each other, and fixing mutual rotation of the rear arm and the front arm in a plane orthogonal to the direction of moving toward and away from each other.

### EFFECT OF THE INVENTION

According to the first aspect, when the arm moving means moves the front arm and the rear arm toward each other, the tip portions of the respective pair of needle-like members can be simultaneously inserted into the corresponding pair of accommodating spaces. Then, the pair of engaging members connected with a suture thread can be simultaneously engaged with the corresponding tip portions of the pair of needle-like members. Accordingly, when tissue such as a stomach wall and the like is merely pinched and released once with the front arm and the rear arm, the suture thread can be passed through the tissue to allow opposite ends (portions connected to the respective engaging members) of the suture thread to be positioned on a rear arm side. Ligating the opposite ends of the suture thread in this state enables the tissue to be sutured in the same manner as suturing in a typical surgical operation.

The second and third aspects each enable tissue large in width and length to be sutured.

The fourth aspect enables a plurality of places to be sutured at the same time, so that suturing work can be performed quickly.

According to the fifth aspect, the tip portions of the respective pair of needle-like members can be reliably accommodated in the corresponding pair of accommodating spaces when the front arm and the rear arm are moved toward each other.

### BRIEF DESCRIPTION OF THE DRAWINGS

Fig. 1 is a schematic perspective view of a suturing device 10 according to the present embodiment in a state of being attached to a shaft 2 of an endoscope 1.
Fig. 2 is a perspective view of the suturing device 10 of the present embodiment in a state of being attached to the shaft 2 of the endoscope 1, as viewed obliquely from below.
Fig. 3 is a perspective view of the suturing device 10 according to the present embodiment in a state of being attached to the shaft 2 of the endoscope 1, as viewed from its rear side.
Fig. 4 is a perspective view of the suturing device 10 itself of the present embodiment.
Fig. 5 is a partial sectional view of the suturing device 10 of the present embodiment.
Fig. 6(A) is a sectional view taken along line VIA-VIA of Fig. 5, and Fig. 6(B) is a sectional view taken along line VIB-VIB of Fig. 5.
Figs. 7(A) and 7(B) are schematic explanatory views of an accommodating portion 17 of a front arm 11 of the present embodiment.
Figs. 8(A) to 8(C) are schematic explanatory views of suturing work with the suturing device 10 of the present embodiment.
Figs. 9(A) to 9(C) are schematic explanatory views of an operation using the suturing device 10 of the present embodiment.

### MODE FOR CARRYING OUT THE INVENTION

Next, embodiments of the present invention will be described with reference to the drawings.

The suturing device of the present invention is characterized in that it is attached to a flexible endoscope to enable suturing from the inside of a gastrointestinal lumen, so that simple and quick suturing can be performed.

The suturing device of the present invention can be used by being attached not only to a flexible endoscope but also to a tip portion of a laparoscope. In particular, when the suturing device of the present invention is attached to a flexible endoscope and used, a full-thickness suture or the like of tissue can be performed even in a state in which no opening or through hole is formed in a gastrointestinal tract within the gastrointestinal lumen. The full-thickness suture means that suturing is performed so as to pass a suture thread through all layers of the gastrointestinal tract (serosa, subserosa, muscularis propria, submucosal layer, and mucosa).

Hereinafter, the case where a suturing device 10 of the present embodiment is attached to a flexible endoscope and used will be described as a representative example.

For easy understanding of structure of each part of the device, relative size of each part in each of the drawings does not necessarily correspond to actual size of the device.

### (Description of Endoscope 1)

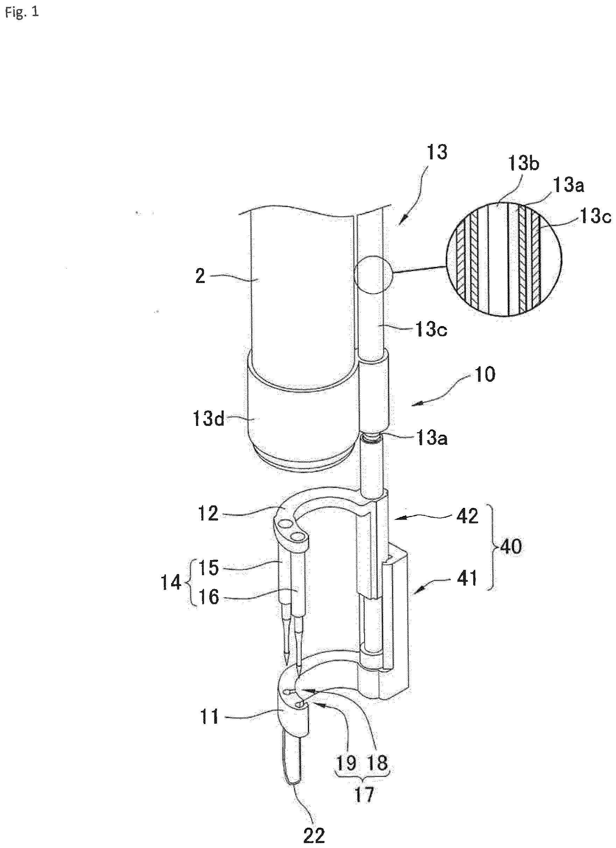

In Fig. 1, reference numeral 2 denotes a shaft of an endoscope 1 to which the suturing device 10 of the present embodiment is attached. The endoscope 1 to which the suturing device 10 of the present embodiment is attached is a flexible endoscope used for general endoscopic surgery. The shaft 2 of the endoscope is not particularly limited in diameter, length, material, and the like, as long as it is used by being inserted into the gastrointestinal tract of a living body. For example, while the shaft 2 has a diameter of about 10 mm for a typical endoscope, it may have a diameter of about 5 mm to 15 mm. While the shaft 2 also has a length of about 1200 mm for a typical endoscope, it may have a length of about 1200 mm to 3000 mm.

### (Description of Suturing Device 10 of Present Embodiment)

As illustrated in Fig. 1, the suturing device 10 of the present embodiment (hereinafter sometimes simply referred to as a suturing device 10) includes a pair of front and rear arms 11 and 12, arm moving means 13 for operating the pair of front and rear arms 11 and 12, and a connecting mechanism 40 for guiding movement of the pair of front and rear arms 11 and 12.

As illustrated in Fig. 1, the suturing device 10 is fixed to the endoscope 1 by fixing the arm moving means 13 to the shaft 2 of the endoscope 1. Specifically, the suturing device 10 is attached such that the axial direction of the arm moving means 13 is substantially parallel to the axial direction of the shaft 2.

The suturing device 10 attached to the shaft 2 as described above enables the arm moving means 13 to reliably follow bending of the shaft 2 when the shaft 2 is bent, for example, so that the arm moving means 13 can be prevented from interfering with the bending or the like of the shaft 2.

In addition, the arm moving means 13 is attached to the shaft 2 of the endoscope 1 such that the pair of front and rear arms 11 and 12 is both positioned in front of a tip surface 2s of the shaft 2 of the endoscope 1, and such that the rear arm 12 is positioned closer to the tip surface 2s of the endoscope 1 with respect to the front arm 11.

As a result, even when the shaft 2 is bent, the pair of front and rear arms 11 and 12 can always be disposed in front of the tip surface 2s of the shaft 2 of the endoscope 1. This enables movement of the pair of front and rear arms 11 and 12 to be visually checked in a reliable manner with a camera provided in the shaft 2. Thus, when tissue in a gastrointestinal tract such as a stomach is sutured using the pair of front and rear arms 11 and 12, operation of the pair of front and rear arms 11 and 12 by an operator can be facilitated.

The arm moving means 13 is not necessarily provided along the shaft 2 of the endoscope 1, and only a tip portion of the arm moving means 13 may be fixed to a tip portion of the shaft 2. Even in this case, operation of the pair of front and rear arms 11 and 12 by an operator can be facilitated as long as the axial direction of the arm moving means 13 and the axial direction of the tip portion of the shaft 2 are substantially parallel to each other in the tip portion of the shaft 2.

Next, each part of the suturing device 10 of the present embodiment will be described.

First, the arm moving means 13 will be described.

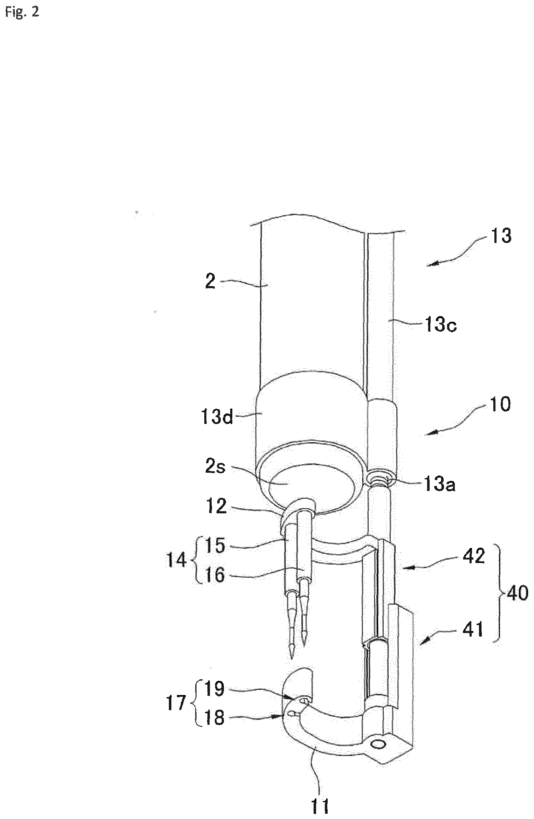

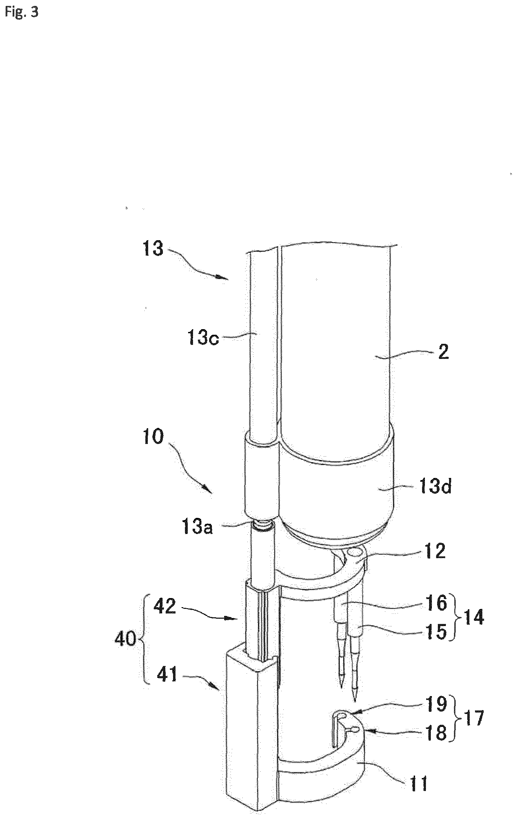

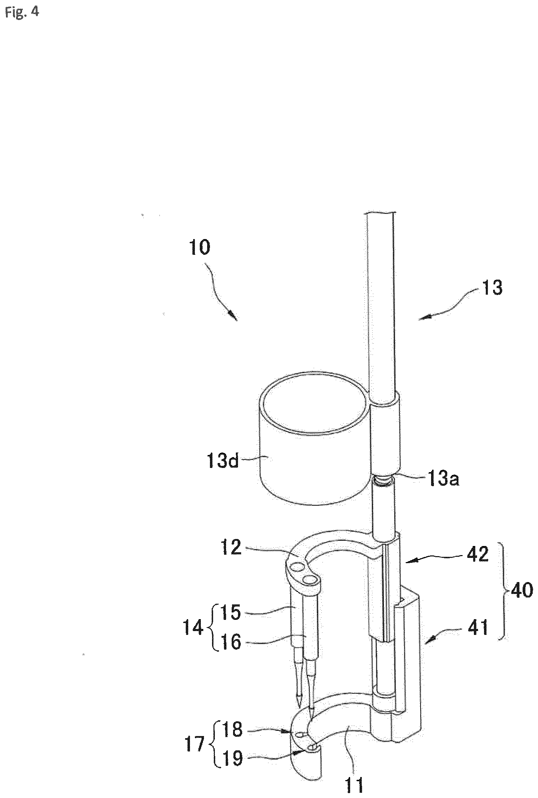

As illustrated in Figs. 1 to 4, the arm moving means 13 is an elongated member extending along its axial direction, and is attached to the shaft 2 of the endoscope 1. The arm moving means 13 have a length that is not particularly limited as long as it is approximately the same as a length of the shaft 2 of the endoscope 1.

The arm moving means 13 has a tip portion provided with a cylindrical fixing portion 13d. When the tip portion of the shaft 2 of the endoscope 1 is inserted into the cylindrical fixing portion 13d, the tip portion of the arm moving means 13 is fixed to the tip portion of the shaft 2 of the endoscope 1.

The arm moving means 13 is fixed such that its portion from the tip portion to a proximal side is along the shaft 2. The arm moving means 13 can be fixed to the shaft 2 with a member such as: a belt-shaped member made of polyethylene, polyvinyl chloride, aluminum, or the like; or a ring-shaped fastener made of polyethylene, polyvinyl chloride, metal, or the like, for example.

The arm moving means 13 is formed of a material having such flexibility that it can be deformed following bending or the like of the shaft 2 while being fixed to the shaft 2. In other words, the arm moving means 13 is formed so as to have such strength that it does not interfere with operation of the endoscope 1 even when being attached to the shaft 2 of the endoscope 1.

A method for fixing the arm moving means 13 to the shaft 2 is not particularly limited, and any method may be used as long as the arm moving means 13 can be fixed so as not to hinder deformation such as bending of the shaft 2.

Specifically, the arm moving means 13 includes a sheath 13a, a wire 13b inserted into the sheath 13a, and a tube 13c into which the sheath 13a is inserted.

The tube 13c is a cylindrical member having a through hole formed from a tip portion of the tube 13c to a proximal end portion thereof, and the tube 13c is fixed to the shaft 2 with a belt-like member or the like, as described above. While material of the tube 13c is not particularly limited, it is preferable that the tube 13c is made of resin such as polyethylene or polyvinyl chloride.

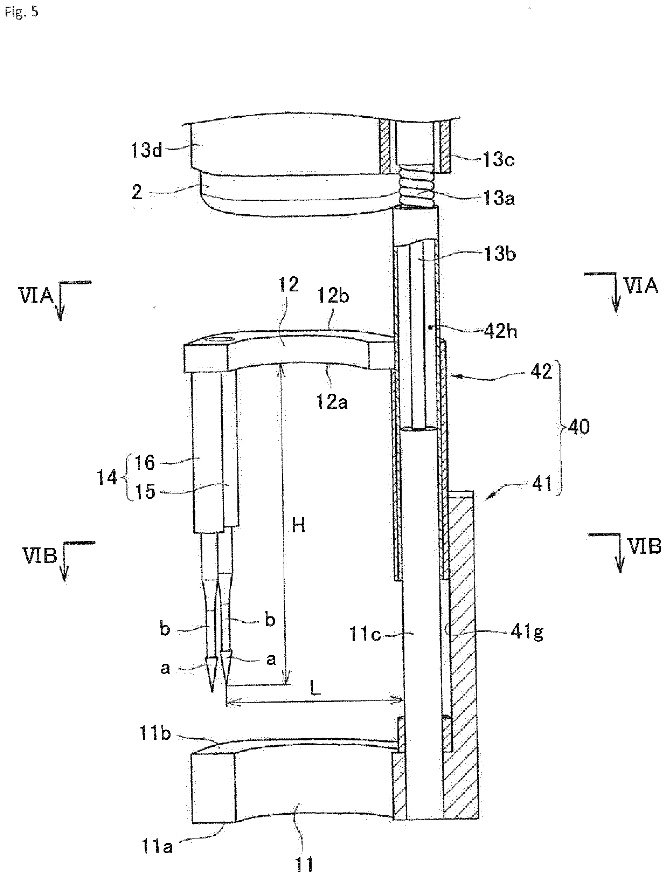

The sheath 13a is inserted into the tube 13c. The sheath 13a is a hollow cylindrical member, and is inserted into the tube 13c so as to be not only movable axially in the tube 13c, but also rotatable in the tube 13c. While material of the sheath 13a is not particularly limited, it is desirable that the sheath 13a is formed so as to have such strength that while the sheath 13a can be deformed following bending of the shaft 2 (or bending of the tube 13c), the sheath 13a is not easily elongated or bent even when an axial force is applied. For example, it is preferable that the sheath 13a is formed of a metal coil tube made of stainless steel or a nickel titanium alloy, a resin tube reinforced with a reinforcing material, or the like. The sheath 13a has a tip fixed to a proximal end of the rear arm 12 (refer to Fig. 5). Meanwhile, the sheath 13a extends such that its proximal end reaches near an operating portion for operating the shaft 2. Thus, when the sheath 13a of the arm moving means 13 is moved to change a position of the proximal end, a length by which the sheath 13a projects from a tip of the tube 13c can be changed. In other words, the rear arm 12 can be moved with respect to the shaft 2 along the axial direction of the shaft 2. In addition, when the sheath 13a is rotated to rotate its proximal end, the rear arm 12 can be rotated around the central axis of the sheath 13a.

The wire 13b is a metal wire or the like, for example, and can be deformed following bending of the shaft 2 (in other words, bending of the sheath 13a). The wire 13b is provided so as to be movable in the sheath 13a along its axial direction. The wire 13b has a tip connected to a proximal end of the front arm 11 (refer to Fig. 5). Meanwhile, the wire 13b extends such that its proximal end reaches near the operating portion for operating the shaft 2. Thus, when the wire 13b of the arm moving means 13 is moved to change a position of the proximal end, a length by which the wire 13b projects from a tip of the sheath 13a can be changed. In other words, the front arm 11 can be moved with respect to the rear arm 12 along the axial direction of the shaft 2.

The structure described above enables the front arm 11 and the rear arm 12 to be moved by operating the proximal end of the sheath 13a and the proximal end of the wire 13b of the arm moving means 13 to move the sheath 13a and the wire 13b along the axial direction. That is, relative positions of the front arm 11 and the rear arm 12 with respect to the tip surface 2s of the shaft 2 can be changed. For example, when the sheath 13a and the wire 13b are moved by the same amount in the same direction, the front arm 11 and the rear arm 12 can be moved in the same direction by the same amount with respect to the tip surface 2s of the shaft 2.

In addition, the sheath 13a and the wire 13b can be independently moved, so that the front arm 11 and the rear arm 12 can be moved toward and away from each other. For example, when movement of the wire 13b is fixed and the sheath 13a is moved, the rear arm 12 can be moved toward and away from the front arm 11. Conversely, when movement of the sheath 13a is fixed and the wire 13b is moved, the front arm 11 can be moved toward and away from the rear arm 12.

An outer diameter of the arm moving means 13 (that is, an outer diameter of the tube 13c) is not particularly limited as long as it is set to such an extent that the endoscope 1 to which the suturing device 10 of the present embodiment is attached can be inserted into a gastrointestinal tract (or an overtube) . For example, the outer diameter of the arm moving means 13 is preferably such that a combined diameter of the outer diameter of the arm moving means 13 and the outer diameter of the shaft 2 is approximately 11 mm to 15 mm, and more preferably approximately 11 mm to 14 mm.

### (Connecting Mechanism 40)

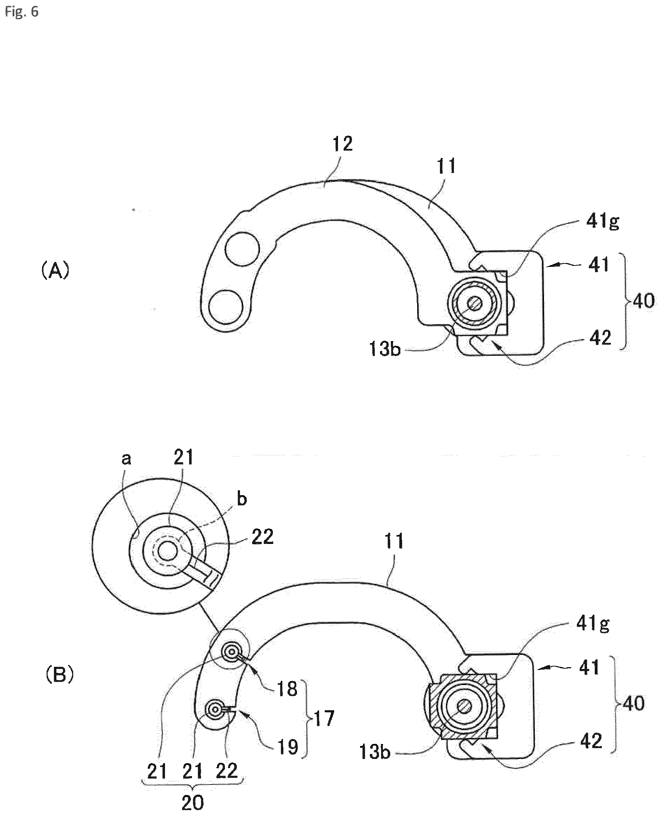

As illustrated in Fig. 5 and Figs. 6 (A) and 6 (B), the pair of front and rear arms 11 and 12 is connected to the tip of the arm moving means 13, and the pair of front and rear arms 11 and 12 is connected by the connecting mechanism 40. That is, the front arm 11 and the rear arm 12 are connected by the connecting mechanism 40 so as to be movable toward and away from each other while mutual rotation of the rear arm and the front arm in a plane orthogonal to the direction of moving toward and away from each other is fixed.

Specifically, the rear arm 12 has a proximal end provided with a rear connecting member 42 formed in a substantially shaft-like shape with a substantially rectangular section of the connecting mechanism 40. The rear connecting member 42 is engaged with a front connecting member 41 provided on the front arm 11. The front connecting member 41 is provided with an engaging groove 41g, and is engaged with the rear connecting member 42 such that three inner faces of the engaging groove 41g are brought into substantially surface contact with the three outer faces of the rear connecting member 42 (or such that a portion in substantially surface contact is formed) (refer to Figs. 6(A) and 6(B)).

The structure described above causes the pair of front and rear arms 11 and 12 to be moved toward and away from each other while the three inner faces of the engaging groove 41g of the front connecting member 41 are in substantially surface contact with the three outer faces of the rear connecting member 42. Thus, when being moved toward and away from each other, the front arm 11 and the rear arm 12 can be moved without allowing the front arm 11 to rotate with respect to the rear arm 12 (or while mutual rotation of the arms in a plane orthogonal to the direction of moving toward and away from each other is fixed).

The connecting mechanism 40 is not limited to the structure described above. The connecting mechanism 40 may have any structure as long as the front arm 11 and the rear arm 12 can be connected to each other so as to be movable toward and away from each other while mutual rotation of the arums in a plane orthogonal to the direction in which both the arms are moved toward and away from each other is fixed. For example, even when not only the front connecting member 41 (or the rear connecting member 42) is provided with a linear groove along the axial direction, but also the rear connecting member 42 (or the front connecting member 41) is provided with a projection to be engaged with the linear groove, a similar effect can be obtained.

### (Description of Pair of Front and Rear Arms 11 and 12)

Next, the pair of front and rear arms 11 and 12 will be described.

### (Rear Arm 12)

First, the rear arm 12 will be described.

As illustrated in Fig. 5, the rear arm 12 has a substantially strip shape with a front face 12a and a rear face 12b that are formed as flat faces parallel to each other. The term "strip shape", as used herein, means a shape with a width shorter than a length and with a thickness also shorter than the length, and has a concept including both of a shape slightly curved along a length direction (refer to Figs. 1 to 4) and a shape extending linearly along the length direction.

The tip of the sheath 13a described above is connected to the proximal end of the rear arm 12. Specifically, the rear connecting member 42 of the connecting mechanism 40 connected to the proximal end portion of the rear arm 12 is connected to the tip of the sheath 13a. The rear arm 12 is connected to the sheath 13a such that the center axis of the sheath 13a is orthogonal to the front face 12a and the rear face 12b of the rear arm 12 at a portion connecting to the sheath 13a. Hereinafter, a direction of the central axis of the sheath 13a at the connecting portion between the sheath 13a and the rear arm 12 may be simply referred to as the central axis of the sheath 13a.

The rear connecting member 42 of the connecting mechanism 40 provided at the proximal end portion of the rear arm 12 is also disposed such that its axial direction is substantially parallel to the central axis of the sheath 13a.

### (Suture Needle Part 14)

Meanwhile, the rear arm 12 has a tip portion provided with a suture needle part 14. The suture needle part 14 includes a pair of needle-like members 15 and 16 disposed side by side parallel to the axial direction of the rear arm 12. Each of the pair of needle-like members 15 and 16 includes a shaft portion b, and a portion (an arrowhead-shaped portion a) provided at a tip of the shaft portion b, having an outer diameter larger than that of the tip. The arrowhead-shaped portion a is formed so as to have an proximal end larger in outer diameter than the tip portion of the shaft portion b, and a step formed at a portion connecting to the shaft portion b. As described below, when the pair of needle-like members 15 and 16 is engaged with a respective pair of engaging members 21 and 21, the arrowhead-shaped portions a serve to prevent the pair of needle-like members 15 and 16 from falling off from the respective pair of engaging members 21 and 21.

The pair of needle-like members 15 and 16 of the suture needle part 14 is each attached to the rear arms 12 such that its tip faces the front arm 11 and its axial direction is orthogonal to the front face 12a of the rear arm 12. In other words, the pair of needle-like members 15 and 16 is each attached to the rear arm 12 such that its central axis is substantially parallel to an axial direction of the rear connecting member 42 (that is, the central axis of the sheath 13a).

### (Front Arm 11)

As illustrated in Fig. 5, the front arm 11 has a substantially strip shape with a front face 11a and a rear face 11b that are formed as flat faces parallel to each other. The front arm 11 is substantially formed in the same shape as the rear arm 12. The front arm 11 has a proximal end portion provided with the front connecting member 41 of the connecting mechanism 40, described above.

The front arm 11 is connected to a tip of the wire 13b. Specifically, the wire 13b is inserted into a through hole 42h passing through the rear connecting member 42 of the rear arm 12, and the tip of the wire 13b is connected to a connecting member 11c provided in a proximal end portion of the front arm 11.

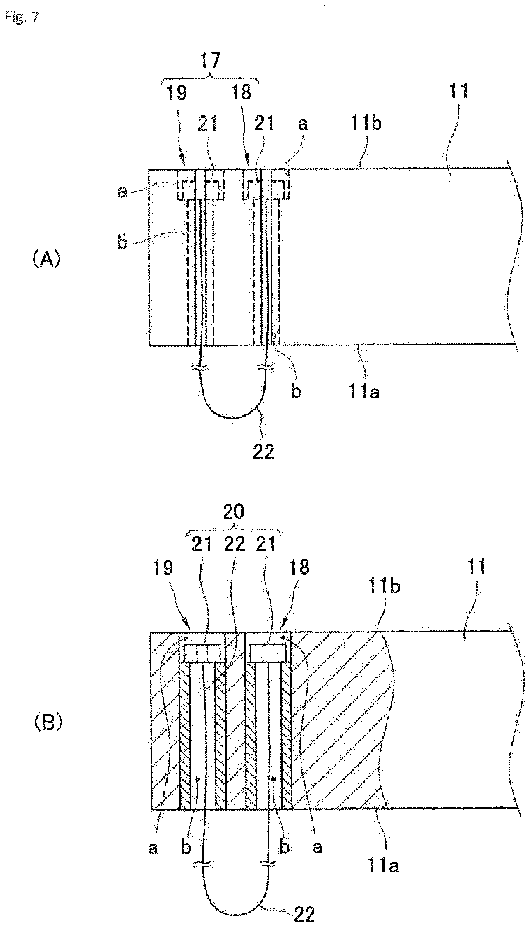

The front arm 11 has a tip portion provided with an accommodating portion 17 having a pair of accommodating spaces 18 and 19 (refer to Fig. 6(B)). As illustrated in Figs. 7(A) and 7(B), the pair of accommodating spaces 18 and 19 of the accommodating portion 17 is each a through hole passing through between the front face 11a and the rear face 11b of the front arm 11. The pair of accommodating spaces 18 and 19 is each formed such that its central axis is parallel to a direction in which the pair of front and rear arms 11 and 12 is moved toward and away from each other (or a direction parallel to the central axis of the sheath 13a). The pair of accommodating spaces 18 and 19 is each provided with an opening communicating with a side face of the front arm 11. The opening allows a suture thread 22 in a suturing instrument 20, described below, to be inserted into the corresponding one of the pair of accommodating spaces 18 and 19 through the opening.

The pair of accommodating spaces 18 and 19 is each a stepped hole having an inner diameter on its rear face 11b side (a large-diameter portion a) larger than an inner diameter on its front face 11a side (a small-diameter portion b). While each of the accommodating spaces 18 and 19 is formed such that the small-diameter portion b has an inner diameter larger than an outer diameter of the arrowhead-shaped portion a, the reason that the accommodating spaces 18 and 19 are formed in such a shape will be described below.

The pair of accommodating spaces 18 and 19 is provided such that their center axes are substantially coaxial with the center axes of the pair of needle-like members 15 and 16, respectively.

The structure described above enables the arrowhead-shaped portions a of the pair of needle-like members 15 and 16 to be inserted into the pair of accommodating spaces 18 and 19, respectively, when the pair of front and rear arms 11 and 12 is moved toward each other.

The central axis of each of the pair of accommodating spaces 18 and 19 is substantially coaxial with the central axis of the corresponding one of the pair of needle-like members 15 and 16. This means that, when the pair of front and rear arms 11 and 12 is moved toward each other, a shift is allowed to the extent that the pair of needle-like members 15 and 16 of the suture needle part 14 can be inserted into the corresponding pair of accommodating spaces 18 and 19.

While the front face and the rear face of each of the front arm 11 and the rear arm 12 are flat in the above example, the front face and the rear face of each of the front arm 11 and the rear arm 12 are not necessarily flat.

In the rear arm 12, a position for providing each of the pair of needle-like members 15 and 16 is not particularly limited. However, as long as the rear arm 12 has a size within a range not interfering with insertion of the rear arm 12 into the body, an advantage that a distance from an end edge of tissue to a suture position can be increased is obtained when each of the pair of needle-like members 15 and 16 is away from the connecting mechanism 40 to some extent. From this viewpoint, a distance L (refer to Fig. 5) from a position of the connecting mechanism 40 (in other words, a position where each of the front arm 11 and the rear arm 12 is connected to the arm moving means 13) to the pair of needle-like members 15 and 16 is preferably at least 5 mm, and more preferably from 10 mm to 20 mm, the distance L being along a plane perpendicular to the axial direction of the arm moving means 13 (in other words, the direction in which the rear arm 12 and the front arm 11 are moved toward and away from each other). The distances L of the respective pair of needle-like members 15 and 16 are equal to each other in the present embodiment. However, when the distances L of the pair of needle-like members 15 and 16 are different from each other, it is preferable that both the distances L are within the range described above.

In addition, the pair of needle-like members 15 and 16 is not particularly limited in material, length, and shaft diameter. Specifically, the pair of needle-like members 15 and 16 may have any length and strength as long as they can pierce an object to be sutured and pass through the object, and can be pulled out from the object by being moved in an opposite direction from a state of having passed through the object. For example, when a stomach wall is sutured by the suturing device 10 of the present embodiment, the pair of needle-like members 15 and 16 may have any length as long as the length from the front face of the rear arm 12 to the tip thereof allows the pair of needle-like members 15 and 16 to pass through the stomach wall, and metal is preferable for the material in terms of strength. More specifically, a length H (refer to Fig. 5) from the tip of each of the pair of needle-like members 15 and 16 to the front face of the rear arm 12 is preferably about 7 mm to 20 mm, and more preferably about 7 mm to 10 mm. The pair of needle-like members 15 and 16 each preferably has a shaft diameter of about 1 mm to 2 mm as a shaft diameter of the tip portion of the shaft portion b (a portion connecting to the arrowhead-shaped portion a), and the arrowhead-shaped portion a preferably has a maximum diameter about 0.1 mm to 1 mm larger than the shaft diameter of the tip portion of the shaft portion b.

### (Suturing Instrument 20)

As illustrated in Fig. 7(B), the suturing device 10 of the present embodiment includes the suturing instrument 20. The suturing instrument 20 includes the pair of engaging members 21 and 21 each formed in an annular shape, and the suture thread 22 connecting the pair of engaging members 21 and 21 to each other.

The pair of engaging members 21 and 21 of the suturing instrument 20 is disposed in the corresponding pair of accommodating spaces 18 and 19 of the accommodating portion 17 of the front arm 11.

When disposed in the corresponding pair of accommodating spaces 18 and 19, the pair of engaging members 21 and 21 is each formed to have a size allowing a through hole passing through two sides of each of the pair of engaging members 21 and 21 to be disposed above a hole passing through the small-diameter portion b of each of the pair of accommodating spaces 18 and 19. Specifically, the pair of engaging members 21 and 21 is each formed to have an outer diameter smaller than the inner diameter of the large-diameter portion a of each of the pair of accommodating spaces 18 and 19, and larger than the inner diameter of the small-diameter portion b thereof (refer to Fig. 7(B)). That is, when the pair of engaging members 21 and 21 is disposed in the corresponding pair of accommodating spaces 18 and 19, the pair of engaging members 21 and 21 is each formed to have a size allowing only a small gap to be formed between an outer edge of each of the pair of engaging members 21 and 21, and an inner face of the large-diameter portion a.

The pair of engaging members 21 and 21 each has a through hole 21h that is formed with a structure in which, while the arrowhead-shaped portion a of the corresponding one of the pair of needle-like members 15 and 16 can be inserted thereinto, the pair of engaging members 21 and 21 does not come off from the corresponding pair of needle-like members 15 and 16 when the arrowhead-shaped portion a completely passes through the through hole 21h. Specifically, the pair of engaging members 21 and 21 is each formed so as to have an inner diameter not only smaller than the outer diameter of the arrowhead-shaped portion a of each of the pair of needle-like members 15 and 16, but also larger than a shaft diameter of the tip (that is, the portion connecting to the arrowhead-shaped portion a) of the shaft portion b of each of the pair of needle-like members 15 and 16.

The material of the pair of engaging members 21 and 21 is not particularly limited. However, it is preferable to use a material having properties capable of maintaining engagement with the pair of needle-like members 15 and 16 while allowing insertion of the arrowhead-shaped portion a of each of the pair of needle-like members 15 and 16, even when the through hole 21h described above is formed. That is, it is desirable that the pair of engaging members 21 and 21 is formed of a material that is elastically deformable to some extent. For example, using a metal plate, a shaft member, or the like enables the pair of engaging members 21 and 21 having the above-described properties to be formed.

### (Outline of Operation of Suturing Device 10 of Present Embodiment)

The structure described above enables the suturing device 10 of the present embodiment to simultaneously insert the pair of needle-like members 15 and 16 into the pair of accommodating spaces 18 and 19, respectively, by operating the arm moving means 13 to move the front arm 11 and the rear arm 12 toward each other.

Then, the arrowhead-shaped portion a of each of the pair of needle-like members 15 and 16 can be simultaneously inserted into the corresponding one of the pair of engaging members 21 and 21 of the suturing instrument 20, disposed in the corresponding pair of accommodating spaces 18 and 19. When the front arm 11 and the rear arm 12 are moved toward each other until the entire arrowhead-shaped portion a of each of the pair of needle-like members 15 and 16 is inserted into the small-diameter portion b of the corresponding one of the pair of accommodating spaces 18 and 19, each of the pair of needle-like members 15 and 16 can be passed through the corresponding one of the pair of engaging members 21 and 21 to its shaft portion b.

When the arm moving means 13 is operated to separate the front arm 11 from the rear arm 12 in this state, the pair of engaging members 21 and 21 can be removed from the corresponding pair of accommodating spaces 18 and 19 together with the corresponding pair of needle-like members 15 and 16. The suture thread 22 can be removed from the pair of accommodating spaces 18 and 19 through the corresponding openings described above.

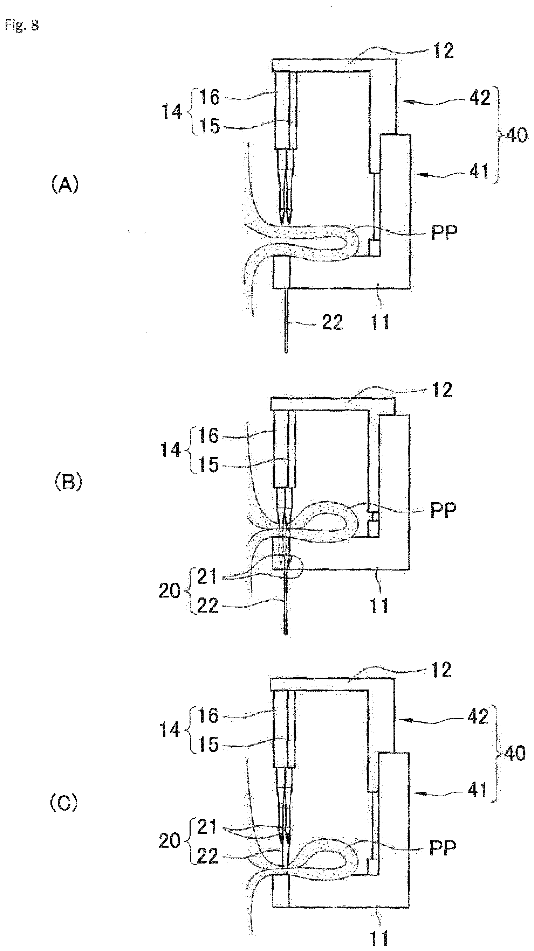

Then, the pair of engaging members 21 and 21 are engaged with the corresponding pair of needle-like members 15 and 16, so that the suture thread 22 connecting the pair of engaging members 21 and 21 can connect the pair of needle-like members 15 and 16 to each other (refer to Fig. 8(C)).

Thus, according to the suturing device 10 of the present embodiment, the suture thread 22 can be passed through an object such that its opposite ends are positioned on the same side of the object by merely moving the front arm 11 and the rear arm 12 once toward and away from each other while the object is disposed between the front arm 11 and the rear arm 12. In other words, the suture thread 22 can be passed through the object such that a portion between the opposite ends of the suture thread 22 is caught by the object (refer to Fig. 8(C)).

### (Suturing of Living Body by Suturing Device 10 of Present Embodiment)

The suturing device 10 of the present embodiment having the structure described above enables tissue such as a stomach wall or the like to be sutured from the inside of the stomach by being attached to the shaft 2 of the endoscope 1.

Hereinafter, suturing work, in which a stomach wall is sutured using the suturing device 10 of the present embodiment while being reversed, will be described with reference to Figs. 8(A) to 8(C). In Figs. 8(A) to 8(C), components are appropriately eliminated to allow movement to be easily understood.

First, the shaft 2 of the endoscope 1 to which the suturing device 10 of the present embodiment is attached is inserted into the stomach. Then, a portion in the stomach wall to be sutured is dented inward by a method of pulling the portion inward with an endoscopic forceps inserted into the endoscope, or the like, to form a protruding portion (protrusion PP) inside the stomach.

In this state, the arm moving means 13 is operated to separate the front arm 11 from the rear arm 12. This forms a gap between the tips of the respective pair of needle-like members 15 and 16 of the suture needle part 14, and the rear face 11b of the front arm 11, the gap allowing the protrusion PP to be pinched. In this state, the shaft 2 is operated to dispose the protrusion PP in the gap between the tips of the respective pair of needle-like members 15 and 16 of the suture needle part 14, and the rear face 11b of the front arm 11 (refer to Fig. 8(A)).

Then, the arm moving means 13 is operated to move the rear arm 12 toward the front arm 11 while movement of the front arm 11 is fixed. This causes the pair of needle-like members 15 and 16 of the suture needle part 14 to pass through the protrusion PP, so that the tips thereof are inserted into the corresponding pair of accommodating spaces 18 and 19. As a result, the arrowhead-shaped portion a of each of the pair of needle-like members 15 and 16 can be engaged with the corresponding one of the pair of engaging members 21 and 21 of the suturing instrument 20 (refer to Fig. 8(B)).

While Fig. 8(B) illustrates the case where the rear arm 12 is moved toward the front arm 11 when the rear arm 12 and the front arm 11 are moved toward each other, the front arm 11 may be moved toward the rear arm 12, or both the arms may be moved toward each other.

When each of the pair of engaging members 21 and 21 is engaged with the arrowhead-shaped portion a of the corresponding one of the pair of needle-like members 15 and 16, the arm moving means 13 is operated to separate the rear arm 12 from the front arm 11. At this time, the pair of needle-like members 15 and 16 returns to their original positions through respective holes that pass through the protrusion PP. This causes both of the pair of engaging members 21 and 21 to which respective opposite ends of the suture thread 22 are fixed to be positioned on one side (an upper side in Fig. 8(C)) of the protrusion PP. As a result, the suture thread 22 forms a loop passing through the protrusion PP from the needle-like member 15 to the opposite side of the needle-like member 15, and then passing through the protrusion PP to return to the needle-like member 16 (refer to Fig. 8(C)).

When the loop of the suture thread 22 is formed as described above, the suture thread 22 is ligated while opposing wall faces of the protrusion PP are brought into contact with each other. Specifically, the suture thread 22 is ligated such that its portion extending from the needle-like member 15 (that is, one engaging member 21) toward the protrusion PP and its portion extending from the needle-like member 16 (that is, the other engaging member 21) toward the protrusion PP are ligated. For this ligation, a commercially available clip and the like can be used. For example, when a clip or the like is supplied from a forceps port of the endoscope 1 and attached to the suture thread 22, the suture thread 22 can be ligated. Alternatively, the suture thread 22 may be ligated by the ligating instrument disclosed in Japanese Patent No. 5294181.

Finally, the suture thread 22 is cut such that its portion positioned closer to the pair of needle-like members 15 and 16 than the ligated portion is cut. This enables the opposing wall faces of the protrusion PP to be fixed while being in contact with each other.

### (Surgery using Suturing Device 10 of Present Embodiment)

When the suturing device 1 of the present embodiment is used, a tumor formed in a stomach wall can be excised without forming an opening in the stomach wall by a method as follows.

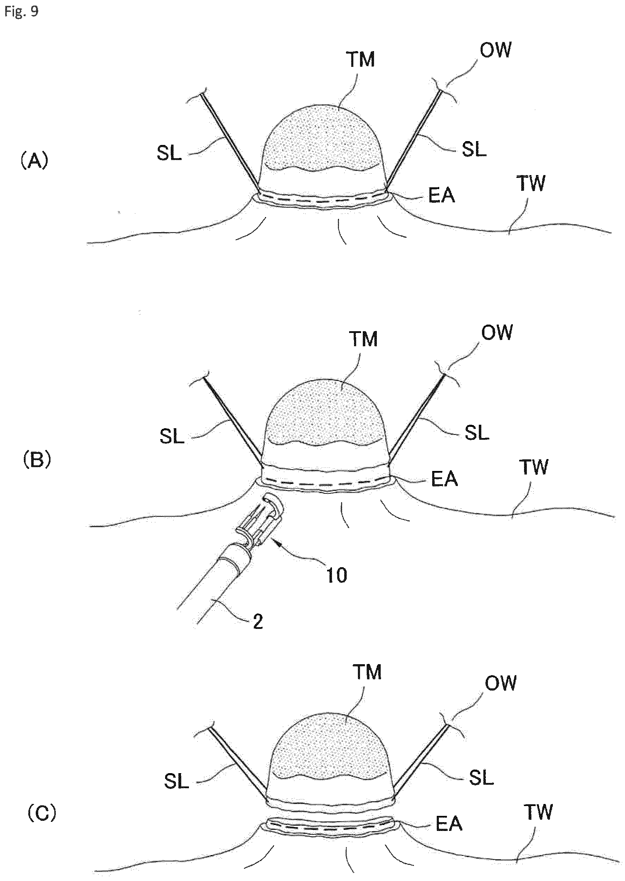

As illustrated in Figs. 9(A) to 9(C), a mucosa and the like around a tumor TM are first excised in a portion where the tumor TM is formed in the stomach wall (a tumor wall TW) to form a portion (an exposed portion EA) where a muscular layer is exposed so as to surround the tumor TM.

Then, the exposed portion EA and a stomach wall (opposing wall OW) facing the portion where the tumor TM is formed are connected by a suture thread SL or the like while the stomach is slightly atrophied. For example, the suturing device disclosed in Japanese Patent No. 5294181 can be used for this connection. The exposed portion EA and the opposing wall OW are connected at at least two places.

After the exposed portion EA and the opposing wall OW are connected, carbon dioxide gas is supplied into the stomach to inflate the stomach. Then, the tumor wall TW and the opposing wall OW are separated from each other. At this time, the exposed portion EA and the opposing wall OW are connected to each other, so that a portion (that is, the tumor TM) surrounded by the exposed portion EA is restricted in inflation as compared with a portion outside the exposed portion EA. That is, the tumor TM is in a state of being raised toward the opposing wall OW by the suture thread SL (Fig. 9(A)).

When the tumor TM is raised, the stomach wall is dented inward while a portion of the tumor TM serves as an apex portion. Then, a position of the exposed portion EA can be sutured by the suturing device 10 of the present embodiment in a manner similar to the suturing of the protrusion PP as described above (refer to Fig. 9(B)).

Then, outer faces of the stomach wall are brought into contact with each other in the sutured portion. As a result, when the exposed portion EA is cut at a position inward of the sutured portion, the tumor TM can be excised (refer to Fig. 9(C)). At this time, the outer faces of the stomach wall is sutured each other in the sutured portion, so that no opening is formed in the stomach wall. That is, the tumor TM and the like can be removed without forming an opening in the stomach wall. As a result, even if the tumor TM is a cancer, seeding of cancer cells into the peritoneal cavity can be prevented.

### (Suture Needle Part 14)

While the suture needle part 14 includes the pair of needle-like members 15 and 16, a distance between the axes of the respective pair of needle-like members 15 and 16 is not particularly limited. The distance may be set to a level allowing a sutured portion to be appropriately sutured with the suture thread 22. For example, when it is set to about 2 mm to 4 mm, a suture state equivalent to suturing in a surgical operation can be achieved.

The above-described example shows the case where the pair of needle-like members 15 and 16 of the suture needle part 14 is disposed side by side so as to be parallel to the axial direction of the rear arm 12. However, the pair of needle-like members 15 and 16 may not necessarily be disposed side by side so as to be parallel to the axial direction. For example, the pair of needle-like members 15 and 16 may be disposed so as to be inclined with respect to the axial direction of the rear arm 12, or may be disposed so as to be orthogonal to each other. The pair of accommodating spaces 18 and 19 of the accommodating portion 17 of the front arm 11 is disposed so as to correspond to the pair of needle-like members 15 and 16 of the suture needle part 14, respectively.

In addition, a plurality of the suture needle parts 14 may be provided side by side parallel to the axial direction of the rear arm 12. In this case, a plurality of portions can be sutured at the same time by moving the front arm 11 and the rear arm 12 once toward and away from each other. As a result, even when a plurality of places need to be sutured, the suturing can be performed in a short time.

For example, when only one suture needle part 14 is provided, it is necessary to replace the suturing device 10 and fill each of the pair of accommodating spaces 18 and 19 of the accommodating portion 17 with the suturing instrument 20 each time suturing is performed once, thereby increasing suture time accordingly.

However, when a plurality of the suture needle parts 14 are disposed side by side parallel to the axial direction of the rear arm 12, a plurality of portions can be sutured at a time, thereby enabling the suturing time to be shortened. Specifically, when two suture needle parts 14 are provided, suturing can be completed in almost half the time as compared with the case where only one suture needle part 14 is provided. This enables a burden on a patient and a doctor who performs an operation to be reduced.

When a plurality of the suture needle parts 14 are provided, the accommodating portions 17 corresponding to the number of the suture needle parts 14 are provided. Then, the accommodating spaces 18 and 19 of each of the accommodating portions 17 are disposed so as to correspond to the pair of needle-like members 15 and 16 of the corresponding one of the suture needle parts 14, respectively.

### INDUSTRIAL APPLICABILITY

The suturing device of the present invention is suitable as an instrument for suturing tissue in a gastrointestinal tract.

### DESCRIPTION OF REFERENCE SIGNS

- 1: endoscope
- 2: shaft
- 10: suturing device
- 11: front arm
- 12: rear arm
- 13: arm moving means
- 14: needle-like part
- 15: needle-like member
- 16: needle-like member
- 17: accommodating portion
- 18: accommodating space
- 19: accommodating space
- 20: suturing instrument
- 21: engaging member
- 22: suture thread
- 40: connecting mechanism
- 41: front connecting member
- 41g: engaging groove
- 42: rear connecting member
- PP: protrusion
- TM: tumor

## Claims

1. A suturing device comprising:
a front arm;
a rear arm provided so as to be movable toward and away from the front arm; and
arm moving means for moving the front arm and the rear arm toward and away from each other,
wherein the front arm and the rear arm each has a proximal end connected to the arm moving means,
the rear arm includes a suture needle part having a pair of needle-like members each provided so as to have a tip portion facing the front arm and a central axis parallel to a direction in which the rear arm and the front arm are moved toward and away from each other,
the front arm includes an accommodating portion provided with a pair of accommodating spaces capable of accommodating the corresponding tip portions of the pair of needle-like members when the front arm and the rear arm are moved toward each other, and
the pair of accommodating spaces of the accommodating portion accommodates a respective pair of engaging members connected to each other with a suture thread and engageable with the corresponding needle-like members.

2. The suturing device according to claim 1, wherein a distance from each of positions at which the respective front arm and rear arm are connected to the arm moving means to the pair of needle-like members is 5 mm or more, the distance being along a face perpendicular to a direction in which the rear arm and the front arm are moved toward and away from each other.

3. The suturing device according to claim 1 or 2, wherein the suture needle part includes the pair of needle-like members provided side by side parallel to an axis of the rear arm, and
the accommodating portion includes the pair of accommodating spaces provided side by side parallel to an axis of the front arm.

4. The suturing device according to claim 1, 2, or 3, wherein
the rear arm includes a plurality of the suture needle parts provided side by side parallel to the axis of the rear arm, and
the front arm includes a plurality of the accommodating portions corresponding to the respective suture needle parts, the plurality of accommodating portions being provided side by side parallel to the axis of the front arm.

5. The suturing device according to claim 1, 2, 3, or 4, comprising a connecting mechanism that connects the rear arm and the front arm while allowing the rear arm and the front arm to move toward and away from each other, and fixing mutual rotation of the rear arm and the front arm in a plane orthogonal to the direction of moving toward and away from each other.
